# EUROPEAN PATENT APPLICATION

(11) **EP 4 756 825 A1**
(43) Date of publication of application: **10.06.2026**
(21) Application number: 25221292.3
(22) Date of filing: 05.12.2025
(51) Int. Cl.: G16H 20/40, G16H 40/63, G16H 40/67

(54) **ADJUSTING AUTOMATED COOPERATIVE OPERATIONS BASED ON SITUATIONALLY DERIVED CONSTRAINTS**

(30) Priority: 06.12.2024 US 202418971596
(71) Applicant: Cilag GmbH International, 6300 Zug (CH)
(72) Inventor: SHELTON, IV, Frederick E., Cincinnati, 45242 (US); HARRIS, Jason L., Cincinnati, 45242 (US); COWPERTHWAIT, Matthew David, Cincinnati, 45242 (US)
(74) Representative: Carpmaels & Ransford LLP

(57) **Abstract**

Systems, methods, and/or instrumentalities disclosed herein may be related to adjusting automated cooperative operations based on situationally derived constraints. A system may be configured to receive a first dataflow and/or a second dataflow from a first surgical element and/or a second surgical element. The second dataflow may indicate a second output parameter associated with a complementary task. The system may determine a relationship between the first surgical element and a second surgical element based on the first dataflow and/or the second dataflow. The system may determine a boundary parameter associated with the second output parameter based on the first output parameter and/or the relationship. The system may send an indication of the boundary parameter to the second surgical element. The system may cause the second surgical element to perform the complementary task based on the boundary parameter.

## Description

### CROSS-REFERENCE TO RELATED APPLICATIONS

This application is related to the following, filed contemporaneously, the contents of each of which are incorporated by reference herein:
- Attorney Docket No. END9638USNP1, entitled PROGRESSIVE ADVANCEMENT OF AUTOMATED LEVEL BASED ON LEARNED COMPLIMENTARY ASSISTANCE
- Attorney Docket No. END9638USNP3, entitled ASSISTANCE ADVANCEMENT MULTI-SYSTEM INTERACTION,
- Attorney Docket No. END9638USNP4, entitled MONITORING AND IDENTIFYING SURGEON CONTROL AND SUGGESTING A TASK THAT MAY BE DONE AUTONOMOUSLY,
- Attorney Docket No. END9638USNP5, entitled CONTROL OF INFORMATION FLOW, PRIORITIZATION AND MANIFESTATION OF DATA ASSOCIATED WITH AN ACTIVE HCP INTERACTION SPACE,
- Attorney Docket No. END9638USNP6, entitled ADAPTIVE RETRACTION FORCE CONTROL,
- Attorney Docket No. END9638USNP7, entitled ADJUSTMENT OR DISPLAY OF OPTIONS OF POSITIONAL OR ORIENTATION IMPLICATIONS ON SURGICAL TOOL USAGE, and
- Attorney Docket No. END9638USNP8, entitled ADJUSTMENT OF PHYSIOLOGIC FUNCTION SUPPLEMENTATION CONTROL.

The contents of each of the following are incorporated by reference herein:
- U.S. Patent Application No. 18/810,323 entitled METHOD FOR MULTI-SYSTEM INTERACTION, filed on August 20, 2024;
- U.S. Patent Application No. 18/960,006 entitled METHOD FOR SMART SURGICAL SYSTEMS filed on November 26, 2024; and
- U.S. Patent Application No. 18/954,186 entitled METHOD FOR MULTI-SYSTEM INTERACTION, filed on November 20, 2024.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

To optimize a task performed autonomously by a second smart device based on an input from a first smart device controlled by a health care professional (HCP), a surgical computing systems may be configured to adjust the automative cooperative operations of a second smart device (e.g., an automated task) based on situationally derived constraints (e.g., a boundary parameter). For example, a surgical computing system may receive a first dataflow from a first smart device that indicates a first output parameter. The output parameter may be associated with a task performed by an HCP (e.g., using the first smart device). The first smart device may be operated manually by an HCP. The surgical computing system may receive a second dataflow from a second smart device, indicating a second output parameter associated with a complementary task. The complementary task may be associated with the first task.

The surgical computing system may determine a relationship between the first smart device and the second smart device based on the first and second dataflows. The surgical computing system may determine a boundary parameter associated with the second smart device. The boundary parameter may indicate a constraint and/or an adjustment to the second smart device during the complementary task. The surgical computing system may determine the boundary parameter based on an output parameter (e.g., of the first and/or second smart device), the determined relationship, and/or a dataflow (e.g., of the first or second smart device) as described herein. The surgical computing system may send the indication of the boundary parameter to the second smart device, to cause the second smart device to perform the complementary task based on the boundary parameter.

Systems, methods, and/or instrumentalities disclosed herein may be related to adjusting automated cooperative operations based on situationally derived constraints. In examples, a system for rendering assistance to a user based on an adaptive recognition of abilities during a procedure may include a processor. The system may be configured to receive a first dataflow from a first surgical element. The first dataflow may indicate a first output parameter associated with a first task. The system may receive a second dataflow from a second surgical element. The second dataflow may indicate a second output parameter associated with a complementary task. The second surgical element may be configured to perform the complementary task based on the first task. The system may determine a relationship between the first surgical element and a second surgical element based on the first dataflow and/or the second dataflow. The relationship may indicate that the complementary task is associated with the first task. The system may determine a boundary parameter associated with the second output parameter based on the first output parameter and the relationship. The boundary parameter may indicate an adjustment to the second surgical element during the complementary task. The system may send an indication of the boundary parameter to the second surgical element. The system may cause the second surgical element to perform the complementary task based on the boundary parameter.

One or more features may be included. For example, the first surgical element may be a first surgical robot configured to be controlled by a user. The first task may be associated with applying radio frequency (RF) energy to tissue of a patient during tissue ablation. The second surgical element may be a second surgical robot configured to be controlled autonomously. The complementary task may be associated with maintaining a tension applied to tissue to secure the tissue during the first task. The boundary parameter may be an energy density applied to the tissue during the tissue ablation. The system may determine the tension to be applied by the second surgical robot during an application RF energy to the tissue by the first surgical robot. The tension to be applied may maintain the energy density applied to the tissue.

The system may receive a physiological parameter of a patient from the first dataflow. The system may determine a second boundary parameter associated with the second output parameter. The system may determine that the physiological parameter of the patient satisfies a threshold. The system may select the second boundary parameter based on a determination that the physiological parameter satisfied the threshold. The system may send an indication of the second boundary parameter to the second surgical element. The system may cause the second surgical element to perform the complementary task based on the boundary parameter and the second boundary parameter. The first surgical element may be configured to execute the first task based on an input from a user. The second surgical element may be configured to execute the complementary task autonomously. The system may determine that the second output parameter satisfies a threshold. The threshold may be associated with the boundary parameter. The system may send an alert message to the first surgical element, wherein the alert message indicates that the output parameter exceeds the boundary parameter. The second surgical element may be configured to perform the complementary task in a synchronized motion based on a movement of the first surgical element while performing the first task. The boundary parameter may be determined further based on a safety risk to a patient during the procedure, historical data, or a quality of service (QoS). The relationship between the first surgical element and the second surgical element may be determined based on a lookup table. The second output parameter may be at least one of: a position, a force, a configuration, or a type of therapy. The system may send the first dataflow, the second dataflow, an indication of the first task, and/or an indication of the complementary task as an input to a machine learning (ML) model. The system may receive, as a response form the ML model, the boundary parameter associated with the second output parameter.

A method for rendering assistance to a user based on an adaptive recognition of abilities during a procedure may include receiving a first dataflow from a first surgical element. The first dataflow may indicate a first output parameter associated with a first task. The method may include receiving a second dataflow from a second surgical element. The second dataflow may indicate a second output parameter associated with a complementary task. The second surgical element may be configured to perform the complementary task based on the first task. The method may include determining a relationship between the first surgical element and a second surgical element based on the first dataflow and the second dataflow. The relationship may indicate that the complementary task is associated with the first task. The method may include determining a boundary parameter associated with the second output parameter based on the first output parameter and/or the relationship. The boundary parameter may indicate an adjustment to the second surgical element during the complementary task. The method may include sending an indication of the boundary parameter to the second surgical element. The method may include causing the second surgical element to perform the complementary task based on the boundary parameter.

In examples, the first surgical element may be a first surgical robot configured to be controlled by a user, the first task may be associated with applying radio frequency (RF) energy to tissue of a patient during tissue ablation, the second surgical element may be a second surgical robot configured to be controlled autonomously, the complementary task may be associated with maintaining a tension applied to tissue to secure the tissue during the first task, the boundary parameter may be an energy density applied to the tissue during the tissue ablation. The method may include determining the tension to be applied by the second surgical robot during an application RF energy to the tissue by the first surgical robot. The tension to be applied may maintain the energy density applied to the tissue.

The method may include receiving a physiological parameter of a patient from the first dataflow. The method may include determining a second boundary parameter associated with the second output parameter. The method may include determining that the physiological parameter of the patient satisfies a threshold. The method may include selecting the second boundary parameter based on a determination that the physiological parameter satisfied the threshold. The method may include sending an indication of the second boundary parameter to the second surgical element. The method may include causing the second surgical element to perform the complementary task based on the boundary parameter and the second boundary parameter.

The first surgical element may be configured to execute the first task based on an input from a user. The second surgical element may be configured to execute the complementary task autonomously. The method may include determining that the second output parameter satisfies a threshold. The threshold may be associated with the boundary parameter. The method may include sending an alert message to the first surgical element. The alert message may indicate that the output parameter exceeds the boundary parameter.

The second surgical element may be configured to perform the complementary task in a synchronized motion based on a movement of the first surgical element while performing the first task. The method may include sending the first dataflow, the second dataflow, an indication of the first task, and/or an indication of the complementary task as an input to a machine learning (ML) model. The method may include receiving, as a response form the ML model, the boundary parameter associated with the second output parameter.

A system for rendering assistance to a user based on an adaptive recognition of abilities during a procedure may be described. The system may include a first surgical element configured to send a first dataflow. The system may include a second surgical element configured to receive a second dataflow. The system receive the first dataflow. The first dataflow may indicate an output parameter associated with a first task and a complementary task. The system may determine a relationship between the first surgical element and the second surgical element. The relationship may indicate that the complementary task is associated with the first task. The system may determine a boundary parameter associated with the second surgical element based on the output parameter and the relationship. The system may send an indication of the boundary parameter to the second surgical element. The system may cause the second surgical element to perform the complementary task based on the boundary parameter.

The system may receive a physiological parameter of a patient from the first dataflow. The system may determine a second boundary parameter associated with the second surgical element. The system may determine that the physiological parameter of the patient satisfies a threshold. The system may select the second boundary parameter based on a determination that the physiological parameter satisfied the threshold. The system may send an indication of the second boundary parameter to the second surgical element. The system may cause the second surgical element to perform the complementary task based on the boundary parameter and the second boundary parameter.

The first surgical element may be configured to execute the first task based on an input from a user, and/or the second surgical element may be configured to execute the complementary task autonomously. The system may send an alert message to the first surgical element. The alert message may indicate that the output parameter exceeds the boundary parameter. The system may send the first dataflow and an indication of the first task as an input to a machine learning (ML) model. The system may receive, as a response form the ML model, the boundary parameter.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system.
FIG. 5 shows an example surgical instrument.
FIG. 6 is an example operational environment in which a surgical computing system may receive dataflow(s) to determine an optimized boundary parameter for a surgical element.
FIG. 7 is a flow chart of an example optimized boundary parameter determination routine performed by a surgical computing system.
FIG. 8A is an example operating environment where a surgical computing device may determine a boundary parameter.
FIG. 8B is a graph illustrating an example boundary parameter that may be utilized by a surgical element to perform a complementary task.

### DETAILED DESCRIPTION

Operating rooms are becoming more sophisticated with the introduction of smart devices (e.g., interchangeably referred to herein as "surgical elements"). Smart devices may be used and/or adjusted by a health care professional (HCP) during a procedure. Smart devices may include one or more advanced capabilities to significantly enhance the precision, safety, and efficiency of a procedure (e.g., a surgical procedure, diagnostic procedure, therapeutic procedure, preventative procedure and/or the like), while reducing the risk of complications to patients. Examples of smart devices may include robotic surgical systems, navigation systems, smart imaging systems, endoscopic and/or laparoscopic systems, harmonic scalpels, anesthesia machines, patient monitoring systems (pulse oximeters, blood pressure monitors, EKG monitors, EEG monitors, and/or the like), energy devices (e.g., electrosurgical units, laser surgery systems, and/or the like), infusion pumps, and/or the like.

Several smart devices may be connected (e.g., via a network) to generate and/or obtain data during a procedure. Examples of data may include real-time data and/or historical data associated with environmental data (e.g., a temperature, humidity, airflow rates, pressure differential, and/or air filtration associated with the operating room), the number and position of HCPs during a procedure, a procedure plan (e.g., patient data, HCP data, tasks, supplies, smart devices, staff workflow and/or the like associated with a procedure), functions of associated smart devices (e.g., sensor data, an output parameter, smart device information, and/or the like), and/or patient data (e.g., physiological parameters, patient health data, and/or the like).

In examples, a first smart device may be controlled by an HCP, and a second smart device may operate autonomously during a procedure. The first and/or second smart devices may perform a complementary task. A complementary task may be a task that is associated with a first task during a procedure. In examples, a complementary task may include a sub-task, a supportive task, a contributing task, a cooperative task, an ancillary task, a supplementary task, and/or a task related to another task. A complementary task may be performed along with a first task to achieve a target outcome. As an illustrative example, a first task may include ablating tissue, while a complementary task may include applying tension to the tissue while the HCP (e.g., via a smart device) ablates the tissue.

A smart device operating autonomously may perform a complementary task, however the smart devices may not determine how to optimally perform the complementary task based on an input from an HCP controlling a first smart device, and/or based on a dataflow generated by the first and/or second smart device. For example, a second smart device (e.g., operating autonomously) may not provide optimized control of the energy density applied to tissue during an ablation procedure based on an HCP operating a first smart device. Energy density may be a summation of the energy output of an ablation device, the force applied to the tissue by the device, and/or a tension of the tissue applied by a second robotic device. The second smart device may not control the output of a tensioner, resulting in an inconsistent energy density at the tissue. This could result in excess damage to non-target tissue and/or insufficient energy to destroy the target tissue. In examples, the second smart device may not be configured to adjust an output parameter based an input received by a first smart device (e.g., based on manual operation of the first smart device by an HCP), to maintain a target energy density.

Additionally, a smart device operating autonomously may not limit or constrain an operation (e.g., a task) based on an input from a first smart device (e.g., operated by an HCP). For example, if an HCP applies excess pressure and/or energy to target tissue, the second smart device may not determine that a limit has been reached for the maximum energy density based on the HCP's actions (e.g., the HCP's manual control of a first smart device), may not generate an alert based on the HCP's actions, may not perform one or more steps to pause ablation to prevent further tissue damage, and/or may not adjust an output parameter to maintain energy density at the target tissue.

To optimize a task performed autonomously by a second smart device based on an input from a first smart device controlled by an HCP, a surgical computing systems may be configured to adjust the automative cooperative operations of a second smart device (e.g., an automated task) based on situationally derived constraints (e.g., a boundary parameter). For example, a surgical computing system may receive a first dataflow from a first smart device that indicates a first output parameter. The output parameter may be associated with a task performed by an HCP (e.g., using the first smart device). The first smart device may be operated manually by an HCP. The surgical computing system may receive a second dataflow from a second smart device, indicating a second output parameter associated with a complementary task. The complementary task may be associated with the first task.

The surgical computing system may determine a relationship between the first smart device and the second smart device based on the first and second dataflows. The surgical computing system may determine a boundary parameter associated with the second smart device. The boundary parameter may indicate a constraint and/or an adjustment to the second smart device during the complementary task. The surgical computing system may determine the boundary parameter based on an output parameter (e.g., of the first and/or second smart device), the determined relationship, and/or a dataflow (e.g., of the first or second smart device) as described herein. The surgical computing system may send the indication of the boundary parameter to the second smart device, to cause the second smart device to perform the complementary task based on the boundary parameter.

As an illustrative example, the first smart device may be a first surgical robot configured to be controlled by an HCP (e.g., operated manually by the HCP), the first task may be associated with applying radio frequency (RF) energy to targeted tissue of a patient during tissue ablation, the second smart device may be a second surgical robot configured to perform a complementary task autonomously, the complementary task may be associated with maintaining a tension applied to tissue, to secure the tissue during the first task. The boundary parameter may be associated with controlling an energy density applied to the target tissue during the tissue ablation. The surgical computing system may determine the tension to be applied by the second surgical robot during an application RF energy to the tissue by the first surgical robot, such that the determined tension maintains the energy density applied to the tissue. Advantageously, the second smart device may optimize a procedure to ablate tissue by contributing and/or compensating for one or more movements of the HCP, to maintain the optimal energy density at the target tissue site.

A more detailed understanding may be had from the following description, given by way of example in conjunction with the accompanying drawings.

### Example Aspects of Surgical Systems

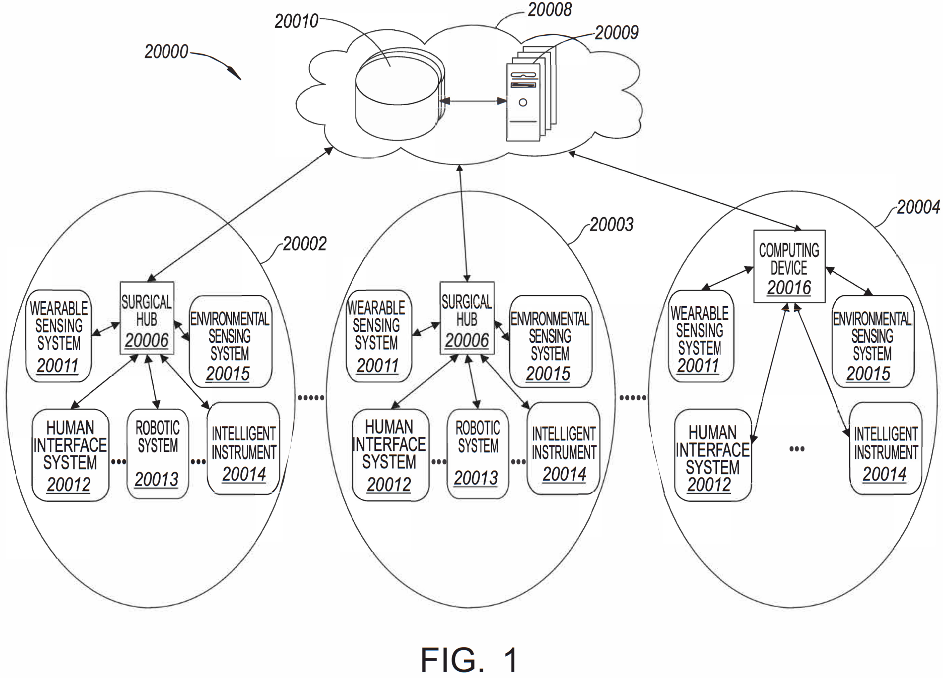

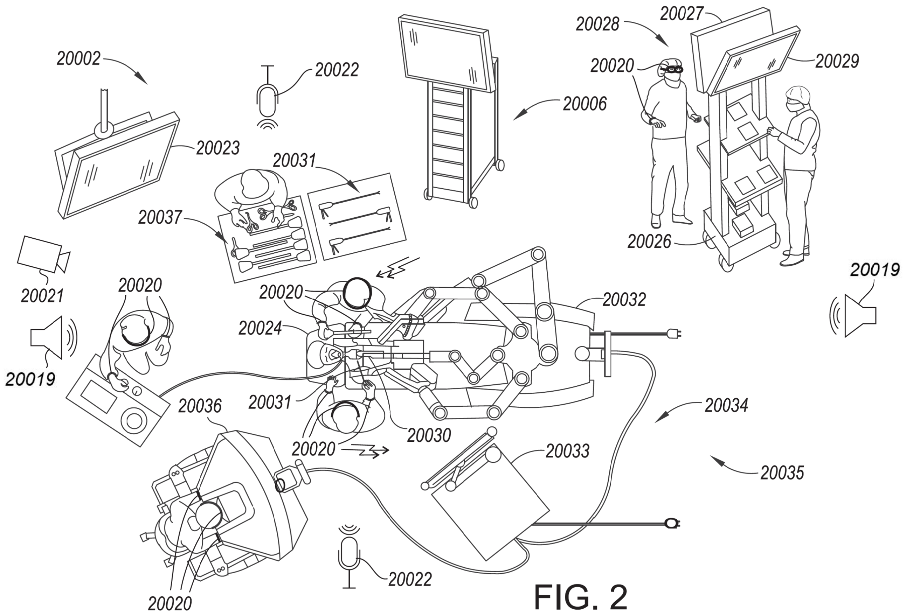

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more HCP sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a HCP. An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

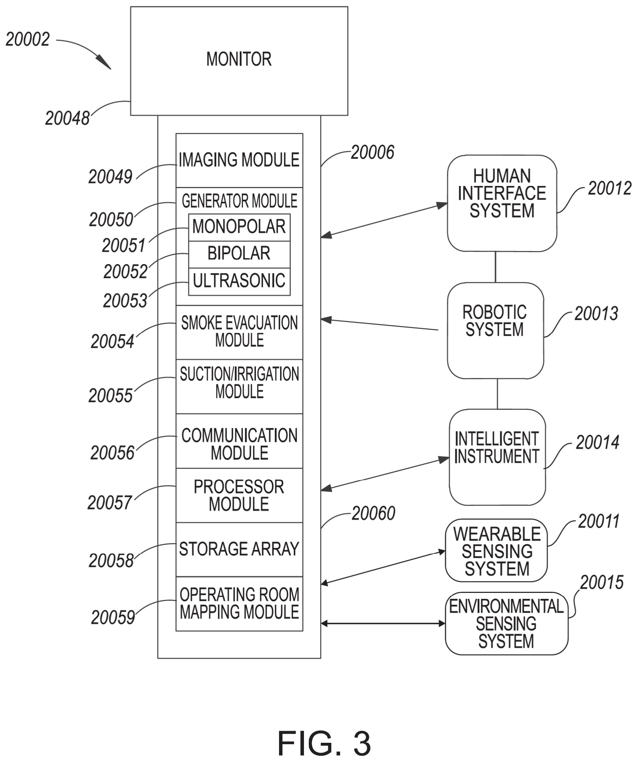

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using, ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

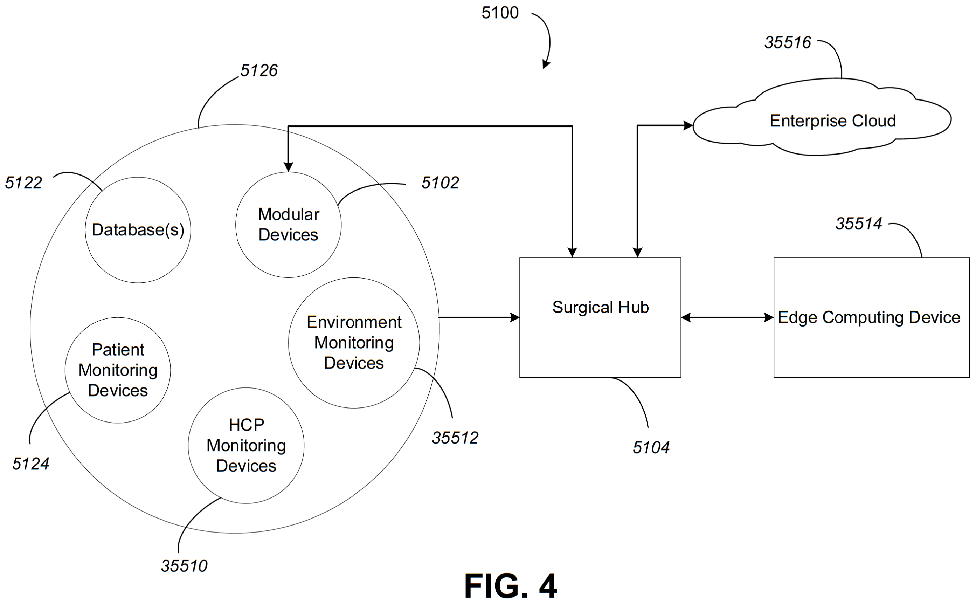

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

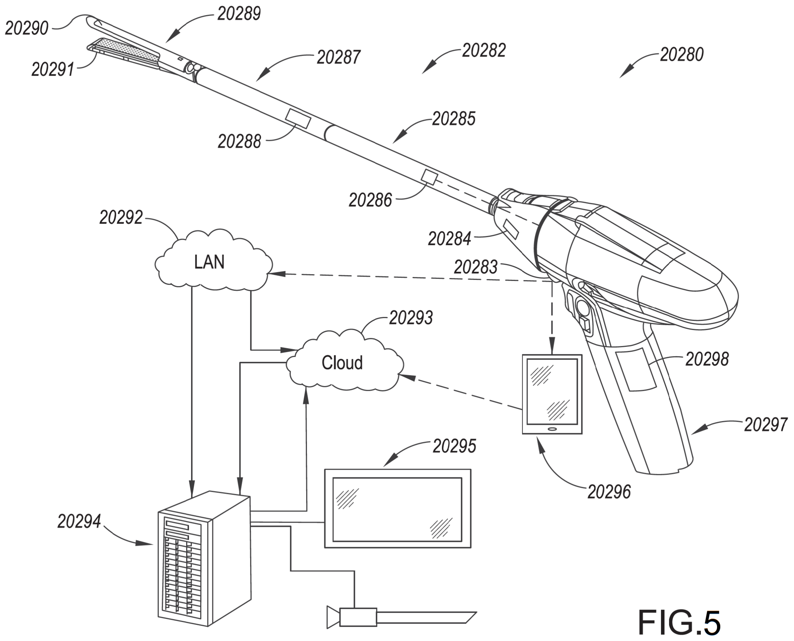

FIG. 5 illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

### Example Aspects of a Surgical Computing System in an Operational Environment

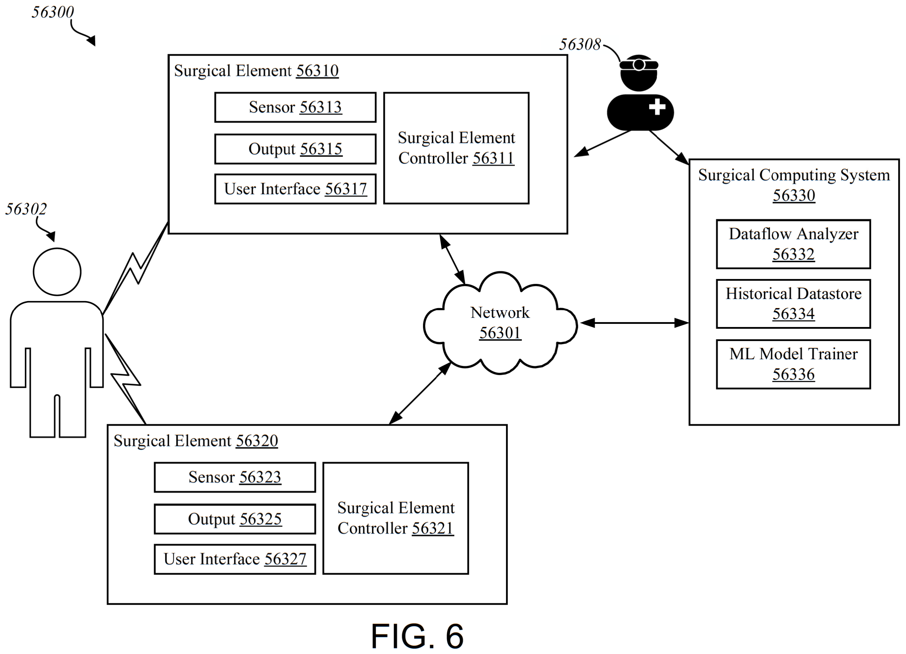

FIG. 6 is an example operational environment 56300. The operational environment 56300 may include a patient 56302, surgical elements 56310, 56320, a system 56330, an HCP 56308, and a network 56301. As part of the operational environment 56300, a surgical computing system 56330 may receive dataflows from surgical element 56310, 56320 via network 56301. The system 56330 may receive dataflows during a procedure on a patient 56302, where an HCP 56308 performs a first task by operating a first surgical element 56310 and/or where a second surgical element 56320 performs a second task (e.g., a complementary task) autonomously, and determine a boundary parameter to optimize the procedure. The system 56330 may send the boundary parameter to the second surgical element 56320, to cause the second surgical element 56320 to optimize the second task associated with a procedure.

An operational environment 56300 may include a surgical computing system 56330 (e.g., referred to hereinafter as "system 56330"). A system 56330 may, among other features, receive a dataflow from surgical element 56310, 56320, via network 56301, and/or a user input from HCP 56308.

A system 56330 may include a dataflow analyzer 56332, historical datastore 56334, and/or ML model trainer 56336. In examples, dataflow analyzer 56332 may generate training data based on historical data received from historical datastore 56334. Dataflow analyzer 56332 may send the training data to ML model trainer 56336, where ML model trainer may train one or more ML models to, among other things, generate a boundary parameter as described herein.

Dataflow analyzer 56332 may be a processor, a microprocessor, a microcontroller, a field programmable gate array (FPGA), an application-specific integrated circuit (ASIC), a system-on-a-chip (SOIC), a digital signal processing (DSP) platform, a real-time computing system, and/or the like. For example, processor may be configured to implement computing functions and/or modules as disclosed herein.

Dataflow analyzer 56332 may transmit and/or receive a dataflow from surgical element 56310, 56320 (e.g., during a procedure). A dataflow may include data generated by and/or associated with the system 56330, a surgical element 56310, 56320, a patient 56302, and/or an HCP 56308. For example, a dataflow may include a boundary parameter, an output parameter (e.g., for controlling output 56315, 56325), data generated by sensor 56313, 56323, data generated by the system 56330, training data, dataflow configuration information, data received from an HCP 56308, and/or historical data as described herein.

In examples, a boundary parameter may indicate an adjustment and/or a constraint on the second smart device during the complementary task. In examples, a boundary parameter may indicate an adjustment to a setpoint, or a constraint on a range of motion (e.g., for a robotic system). In examples, a boundary parameter may indicate an automation level (e.g., a quantity of tasks that may be automated by a second surgical element 56320 for a procedure). In examples, a boundary parameter may indicate a mapped path that a second (e.g., autonomous) surgical element 56320 may traverse. The mapped path may be determined based on an HCP 56308 while manually operating a first surgical element 56310.

An output parameter may control the output of a surgical element 56310, 56320. In examples, an output parameter may indicate a control variable setpoint (e.g., associated with a motor speed, a temperature, a flow rate, and/or the like), a current and/or voltage (e.g., associated with an electrosurgical tool, and/or the like), an instruction (e.g., to generate an image from a camera and/or the like), and/or a power level (e.g., of a heating pad, a laser ablation tool, and/or the like). As an illustrative example, a output parameter may include a setpoint for the speed of a cutting tool, a voltage and/or current setpoint of an electrosurgical tool, a flow rate for an infusion pump, a position of a robotic arm, and/or the like.

In examples data generated by sensor 56313, 56323, may include a signal indicating one or more physiological parameters of a patient (e.g., oxygen saturation, blood pressure, respiratory rate, blood sugar, heart rate, a core body temperature, a hydration state and/or the like), an environmental characteristic of the operating room (e.g., a room temperature, a number of HCPs during a procedure, the position of HCPs during a procedure, a humidity level, air quality, lighting levels, CO2 levels and/or the like) and/or one or more characteristics of surgical element 56310, 56320 (e.g., a current, voltage, an pressure, a force applied, a distance, a vibration, an orientation, a flow rate, a status, and/or the like). As an illustrative example an HVAC system may include a temperature, humidity, and/or an air quality sensor. In examples, a robotic surgical system may include a force and/or pressure sensor, a depth sensor, a temperature sensor, a blood flow and/or oxygen sensor, a pH sensor (e.g., to measure blood gas), an electrocardiogram (ECG) sensor, a position sensor (e.g., to measure the position of a robotic arm), and/or the like.

In examples, data generated by the system 56330 may include a procedure plan, morbidity data associated with one or more patients, an HCP 56308 skill level, preferred tools used by an HCP 56308, a task, supplies, whether a surgical element 56310, 56320 is available for a procedure, and/or staff workflow (e.g., number of HCP(s) 56308 and/or surgical elements 56310, 56320, necessary for a task). Data generated by the system 56330 may include an indication of a relationship (e.g., between a first surgical element 56310 and a second surgical element 56320), an indication of a task and/or complementary task that may be automated, a threshold associated with a physiological parameter of a patient (e.g., a life-threatening threshold such as a core-body temperature limit, a SpO2 limit, and/or the like), and/or a threshold associated with a boundary parameter (e.g., an area outside a mapped path that a surgical element 56310, 56320, is instructed to traverse).

In examples, training data may include an indication of a boundary parameter, an output parameter, data generated by sensor 56313, 56323, data generated by the system 56330, data received by an HCP 56308, historical data, and/or dataflow configuration information used to train an ML model. The dataflow analyzer 56332 may generate and/or receive training data from surgical element 56310, 56320. Dataflow analyzer 56332 may send training data to ML model trainer 56336 to, among other things, determine a relationship between surgical elements 56310, 56320, and/or determine a boundary parameter as described herein.

In examples, dataflow configuration information may indicate surgical element ID, whether a dataflow is available, a unit of measure, a communication protocol (RS-232, Ethernet, TCP/IP, Bluetooth, and/or the like), scheduling and/or frequency information (e.g., a time and/or frequency that sensor data is to be sent), a destination (e.g., port information associated with the surgical element controller 56311, 56321, and/or the system 56330), and/or security and/or access control credentials. In examples dataflow configuration information may be used to configure a system 56330 to receive an output parameter, a boundary parameter, sensor data, and/or the like from a first and/or second surgical element 56310, 56320.

In examples, historical data may include past information associated with a procedure. For example, historical data may include a dataflow (e.g., a boundary parameter, an output parameter, data generated by sensor 56313, 56323, data generated by the system 56330, training data, dataflow configuration information, data received by an HCP 56308, and/or the like). Data analyzer 65332 may use historical data to, among other things, determine a relationship between surgical elements 56310, 56320.

In examples, data received from an HCP 56308 may include an indication of a procedure, a task, a complementary task, a relationship, a threshold (e.g., associated with a boundary parameter, a life-threatening threshold, and/or the like), an instruction to automate a task, and/or the like. Dataflow analyzer 56332 may receive a user input from and/or generate an output to an HCP 56308 (e.g., via user interface 56317, 56327). In examples, dataflow analyzer 56332 may send an indication of an alert to the HCP 56308. The alert may be generated in response to a determination that an output parameter satisfies a threshold (e.g., associated with a boundary parameter and/or a physiological parameter of the patient 56302). In examples, dataflow analyzer 56332 may receive a user input form an HCP 56308, including an indication of a relationship between surgical element 56310 and 56320, a procedure, a task, a complementary task, and/or the like (e.g., to be performed autonomously by a surgical element 56310, 56320).

The dataflow analyzer 56332 may determine a complementary task that may be automated and then allow the user (e.g., an HCP 56308 via a user interface 56317) to select whether to automate the task. In examples, the HCP 56308 may select to manually perform an indicated task, and/or request that the system 56330 obtain additional data (e.g., via a dataflow) based on execution of the manual task. In examples, the system 56330 may receive acknowledgement that a first task is successfully automated before determining a second task to automate. As an illustrative example, the system 56330 may perform suturing numerous times to develop a basic level of competency in suturing (e.g., as determined by the HCP 56308). The system 56330 may indicate the corresponding elements of arm positioning, etc., before determining a second automated task.

The system 56330 may provide automation levels (e.g., as indicated in an automation assistance parameter, a determined number of tasks that may be automated by one or more surgical elements 56310, 56320) associated with supporting an HCP 56308 during a procedure based on the aspect of the surgeon. For example, for opening and closing a patient 56302, a robotic system (e.g., a surgical element 56320) may provide a lower level of automation. However, for laparoscopic dissection within the procedure, the robotic system may provide a higher level of automation (e.g., the system 56330 may determine a second automation assistance parameter based on one or more tasks associated with a procedure), and then for stapling actions, the robotic system may provide a lower level of support.

In examples, the automation assistance parameter may indicate instructions associated with routine tasks and/or complex tasks. For example, in suturing, an automation assistance parameter may indicate simple suturing scenarios, as well as more complicated suturing scenarios based on tissue, anatomical access, and other constraints (e.g., based on operational data).

Dataflow analyzer 56332 may determine and/or infer one or more relationships (e.g., determine a relational link) between components of an operational environment 56300. In examples, dataflow analyzer 56332 may determine a relationship between surgical elements 56310, 56320. A relationship may be determined and/or inferred based on, for example, compatible sensors (e.g., sensor 56313, 56323) between surgical elements 56310, 56320, a determined dataflow that may be needed to resolve an issue during a procedure, and/or based on a user input (e.g., by an HCP 56308 via a user interface 56317, 56327). As an illustrative example, a dataflow analyzer 56332 may receive a first dataflow from a laparoscopic tool (e.g., a dataflow indicating a measured temperature of an insufflated cavity via sensor 56313) and a second dataflow from a heating pad (e.g., a dataflow indicating a measured temperature near an insufflated cavity via sensor 56323). The dataflow analyzer 56332 may determine, based on configuration information, sensor data, and/or an output parameter associated with each dataflow, that the two dataflows are related (e.g., a change detected by a first temperature sensor of a second surgical element 56320 may be related to a response and/or a change in an output parameter for a first surgical element 56310). In examples, sensor data may be related if, for example, the sensor data from a first and second surgical element 56310, 56320, share a similar unit of measure, measure a similar physiological parameter of a patient's body, are proximate to one another during a procedure, and/or the like. After the dataflow analyzer 56332 determines that one or more aspects of surgical elements 56310, 56320 are related, the dataflow analyzer 56332 may store an indication of the relationship in, for example, historical datastore 56334 (e.g., in a look-up table (LUT) defining the one or more relationships). Additionally and/or alternatively, dataflow analyzer 56332 provide training data (e.g., including historical data) to ML model trainer 56336, to train an ML model based on one or more relationships.

In examples, dataflow analyzer 56332 may determine that a relationship exists based on dataflow configuration information (e.g., based on a surgical element ID, a communication protocol, scheduling and frequency information, a destination, security and access control credentials, and/or a unit of measure), for a surgical elements 56310, 56320. As an illustrative example, a dataflow analyzer 56332 may receive a dataflow from a laparoscopic tool (e.g., a dataflow indicating a measured temperature of an insufflated cavity via sensor 56313) and a dataflow from a heating pad (e.g., a dataflow indicating a measured temperature near an insufflated cavity via sensor 56323 and/or a power level associated with heat generated by the heating pad (e.g., via output 56325)). Dataflow analyzer 56332 may determine that an increase in temperature, as measured by the laparoscopic tool, is caused by the power output associated with the heating pad. Dataflow analyzer 56332 may indicate that the temperature sensor associated with the laparoscopic tool may be used, among other things, to control the power output of the heating pad.

Dataflow analyzer 56332 may determine a boundary parameter associated with an output parameter (e.g., of a surgical element 56310, 56320). A boundary parameter may indicate a spatial boundary, a force, a speed and/or acceleration, a proximity, a range of motion, a tissue type, an image, a user controlled limit, and/or the like. As an illustrative example, a boundary parameter (e.g., a spatial boundary parameter) may enable a second surgical element 56320 to maintain a safe distance to avoid interference or a collision with a first surgical element 56310. A boundary parameter (e.g., a force boundary parameter) may limit excessive pressure and/or strain on tissue to prevent tissue damage, and/or to maintain a target energy density (e.g., during a moving ablation procedure). A speed and/or acceleration boundary may limit how quickly a surgical device may move (e.g., limit an output parameter such as the speed of a motor while moving through high-risk areas). A proximity boundary parameter may limit motion to prevent unintentional contact with the patient 56302, surgical tools, an HCP 56308, and/or the like. A tissue-type boundary parameter may limit an automated task (e.g., a complementary task) based on a tissue characteristic (e.g., based on tissue density, elasticity, high-vascularity tissue, and/or how delicate the tissue is).

A dataflow analyzer 56332 may determine a second boundary parameter based on a first boundary parameter. As an illustrative example, a dataflow analyzer 56332 may determine a tissue-type boundary parameter (e.g., a first boundary parameter associated with tissue density, elasticity, high-vascularity, or how delicate the tissue is). The tissue-type boundary parameter may be used to determine a second boundary parameter. The second boundary parameter may be a spatial boundary parameter and/or a velocity boundary parameter (e.g., to prevent unintentional contact or reduce the likelihood of tissue damage). As an example, a dataflow analyzer 56332 may use an image boundary parameter to adjust a spatial boundary parameter and/or adjust a range of motion (e.g., to limit the movement of a catheter and/or the like).

In examples, a boundary parameter may be associated with an operating envelope for a second surgical element 56320. The boundary parameter may indicate an adjustment to an operating envelope of a second surgical element 56320 based on an action associated with a first surgical element (e.g., a movement of a first surgical element 56310 when controlled by an HCP 56308 and/or a user input from an HCP 56308 via user interface 56317, 56327).

In examples, a boundary parameter may include a safety envelope. A dataflow analyzer 56332 may determine a safety envelope for a patient 56302 based on a dataflow, historical data, and/or a user input from an HCP 56308. In examples a boundary parameter may be determined based on safety risk to a patient during the procedure, historical data, or a quality of service (QoS). For example, dataflow analyzer 56332 may determine that a task and/or a complementary task may be performed during a first time based on a determined boundary parameter (e.g., the task and/or complementary task may be performed within a time period associated with a determined safety envelope). As an illustrative example, a dataflow analyzer 56332 may determine a blood pressure range. A task and/or a complementary task may be performed while the patient's blood pressure is within the blood pressure range (e.g., the safety envelope). In examples, for patient's susceptible to hypothermia, a dataflow analyzer 56332 may determine a safety envelope that may maintain a body temperature within a range during a task and/or complementary task. In examples, if the safety envelope threshold is satisfied (e.g., a parameter falls outside a range and/or the like), the dataflow analyzer 56332 may determine a boundary parameter that reduces an action associated with a complementary task, increases the level of automation to fully automate a task, stop a complementary task, and/or alert the HCP 56308 (e.g., via user interface 56317, 56327).

A dataflow analyzer 56332 may determine a boundary parameter based on a complementary task. A complementary task may be performed by a second surgical element 56320 based on a first task performed by a first surgical element 56310 (e.g., during a procedure). As described herein, a complementary task may be a sub-task, a supportive task, a contributing task, a cooperative task, an ancillary task, a supplementary task, and/or a task related to another task. A complementary task may be performed along with a first task to achieve a target outcome. As an illustrative example, a first task may include applying energy to ablate tissue (e.g., via a first surgical element 56310), while a complementary task may include applying tension to the tissue (e.g., via a second surgical element 56320 operating autonomously) to achieve a target energy density during the application of energy. In examples, the boundary parameter may include an instruction to perform a complementary task in a synchronized motion based on a movement of a first surgical element 56310 (e.g., while the first surgical element 56310 performs a first task). For example, the boundary parameter may instruct the second surgical element 56320 to vary an amount of force applied to tissue relative to the amount of force applied by the HCP 56308 (e.g., via the first surgical element 56310) and/or relative to the energy output determined by the HCP 56308, to achieve a target energy density.

Dataflow analyzer 56332 may determine a boundary parameter based on a physiological parameter of a patient (e.g., received from surgical element 56310, 56320). For example, the dataflow analyzer 56332 may determine a boundary parameter based on a perfusion level, an electrophysiological signal, a blood pressure, a heart rate, a respiratory rate, the temperature of tissue, SpO2, CO2 levels, electroencephalogram (EEG) readings, and/or the like. As an illustrative example, dataflow analyzer 56332 may receive a dataflow including an indication of a tissue temperature, and determine a boundary parameter that limits the movement of a second surgical element 56320 during ablation (e.g., if a tissue's temperature exceeds a threshold, the boundary parameter may limit a robot's movement proximate to the tissue). As a second illustrative example, if a patient's SpO2 level satisfies a threshold, dataflow analyzer 56332 may determine a boundary parameter that limits a robot's movement in proximity to the patient's lungs and/or airways.

Dataflow analyzer 56332 may transmit an alert to surgical element 56310, 56320. Dataflow analyzer 56332 may generate an alert if at least one of a physiological parameter, a boundary parameter, or an output parameter satisfies a threshold. In examples, dataflow analyzer 56332 may generate an alert if an HCP 56308 (e.g., via a first surgical element 56310) performs an action that causes an output parameter (e.g., of the first or second surgical element 56310, 56320), to satisfy a threshold. As an illustrative example, dataflow analyzer 56332 may generate an alert if an HCP 56308 applies an excessive amount of energy during a moving ablation procedure, such that the second surgical element 56320 is not able to move and/or apply force to tissue sufficient to maintain a target energy density (e.g., due to a boundary parameter limiting the speed of the second surgical element 56320 to prevent damage to tissue).

Historical datastore 56334 may store and/or include data associated with one or more past procedures. Historical datastore 56334 may store data generated by surgical elements 56310, 56320, an HCP 56308 (e.g., via a user interface 56317, 56327), and/or by the system 56330.

In examples, historical datastore 56334 may store a boundary parameter, an output parameter (e.g., for controlling output 56315, 56325), data generated by sensor 56313, 56323, data generated by the system 56330, training data, dataflow configuration information, data received by an HCP 56308, and/or historical data. Historical datastore 56334 may store an ML model (e.g., generated by ML model trainer 56336). In examples, historical datastore 56334 may store data used by the dataflow analyzer 56332 to determine one or more relationships associated with surgical elements 56310, 56320. In examples, data stored in historical datastore 56334 may include a LUT that indicates one or more relationships between surgical elements 56310, 56320. As an illustrative example, historical datastore 56334 may store information associated with identifying relationships between dataflows (e.g., based on dataflow configuration information), such as a surgical element ID, a communication protocol, scheduling and frequency information, a destination, security and access control credentials, a unit of measure, and/or the like.

A system 56330 may include ML model trainer 56336. ML model trainer 56336 may receive training data from dataflow analyzer 56332. Training data may include information associated with a dataflow (e.g., a boundary parameter, an output parameter, data generated by sensor 56313, 56323, data generated by the system 56330, data received by an HCP 56308, historical data, and/or dataflow configuration information. In response to receiving training data, ML model trainer 56336 may train an ML model to, among other things, determine a relationship between surgical elements 56310, 56320, and/or determine a boundary parameter as described herein.

ML model trainer 56336 may train an ML model to determine a relational link (e.g., a relationship) associated with a first surgical element 56310 and/or a second surgical element 56320. A relational link may be determined in real-time (e.g., during a procedure), or at another time (e.g., based on data stored in historical datastore 56334 as described herein). A relationship may be determined based on one or more aspects of a surgical element 56310, 56320 (e.g., information associated with a dataflow, historical data, configuration information, and/or the like). ML model trainer 56336 may store an indication that one or more aspects of surgical elements 56310, 56320 are related in historical datastore 56334 (e.g., via a LUT).

In examples, ML model trainer 56336 may train an ML model to determine that a relationship exists between sensor data associated with a first surgical element 56310 (e.g., sensor 56313) and sensor data associated with a second surgical element 56320 (e.g., sensor 56323). As described herein, sensor data may be related if, for example, sensor data shares a similar unit of measure, the sensor data measures a similar physiological parameter of a patient's body, the sensors are proximate to one another during a procedure, and/or the like.

In examples, ML model trainer 55363 may train an ML model to determine that a relationship exists between sensor data associated with a first surgical element 56310 (e.g., sensor 56313) and/or an output parameter generated for a second surgical element 56320 (e.g., an output parameter used to adjust the output 56325). Sensor data associated with a first surgical element 56310 (e.g., sensor 56313) may be related to an output parameter generated for a second surgical element 56320 if, for example, a sensor 56313 measures an output characteristic associated with the second surgical element 56320 as described herein.

In examples, ML model trainer 56336 may train an ML model to determine that a relationship exists based on dataflow configuration information (e.g., based on a surgical element ID, a communication protocol, scheduling and frequency information, a destination, security and access control credentials, and/or a unit of measure, and/or the like), for a surgical elements 56310, 56320. As an illustrative example, a dataflow analyzer 56332 may transmit to ML model trainer 56336, a first dataflow from a laparoscopic tool (e.g., a dataflow indicating a measured temperature of an insufflated cavity via sensor 56313) and a second dataflow from a heating pad (e.g., a dataflow indicating a measured temperature near an insufflated cavity via sensor 56323 and/or a power level associated with heat generated by the heating pad (e.g., via output 56315)). ML model trainer 56336 may determine that an increase in temperature, as measured by the laparoscopic tool, is caused by the power output associated with the heating pad. An ML model trained by ML model trainer 56336 may indicate, to the dataflow analyzer 56332, that the temperature sensor associated with the laparoscopic tool may be used to control the power output of the heating pad (e.g., a relationship between the first and second surgical element 56310, 56320).

ML model trainer 56336 may train an ML model to determine a boundary parameter. As described herein, a boundary parameter may indicate a spatial boundary, a force, a speed and/or acceleration, a proximity, a range of motion, a tissue type, an image, a user controlled limit, and/or the like. An ML model trainer 56336 may train an ML model to determine one or more boundary parameters based on dataflow(s), historical data, a complementary task, a physiological parameter of a patient, a first boundary parameter, and/or the like.

As an illustrative example, an ML model trainer 56336 may train an ML model to determine a tissue-type boundary parameter (e.g., associated with tissue density, elasticity, high-vascularity, or how delicate the tissue is), and determine a spatial boundary parameter and/or a velocity boundary parameter (e.g., to prevent unintentional contact or reduce the likelihood of tissue damage). An ML model may receive an image as an input to an ML model, and adjust a spatial boundary parameter and/or adjust a range of motion (e.g., to limit the movement of a catheter and/or the like).

ML model trainer 56336 may train an ML model to determine an operating envelope for a second surgical element 56320. The ML model may output a boundary parameter may indicating an adjustment to an operating envelope of a second surgical element 56320 based on an input including an action associated with a first surgical element 56310 (e.g., a movement of a first surgical element 56310 when controlled by an HCP 56308 and/or a user input from an HCP 56308 via user interface 56317, 56327).

ML model trainer 56336 may train an ML model to determine a boundary parameter associated with a safety envelope. For example, an ML model may be trained to determine that a task and/or a complementary task may be performed during a first time based on a determined boundary parameter (e.g., the task and/or complementary task may be performed within a time period associated with a determined safety envelope). As an illustrative example, an ML model trainer 56336 may train an ML model to determine a blood pressure range (e.g., a safety envelope). A task and/or a complementary task may be performed while the patient's blood pressure is within the blood pressure range (e.g., the safety envelope determined by the ML model). In examples, for patient's susceptible to hypothermia, an ML model trainer 56336 may train an ML model to determine a safety envelope that maintains a body temperature within a range during a task and/or complementary task. In examples, an ML model may be trained to determine a boundary parameter that reduces an action associated with a complementary task, increases the level of automation to fully automate a task, and/or stop a complementary task and alert the HCP 56308.

An operational environment 56300 may include surgical elements 56310, 56320. Although two surgical elements are included as part of the operational environment 56300, this is not meant to be limiting as multiple surgical elements may be included and/or in communication with one or more components of the operational environment 56300. Surgical element 56310 may include surgical element controller 56311, sensor 56313, output 56315, and/or user interface 56317. Surgical element 56320 may include surgical element controller 56321, sensor 56323, output 56325, and/or user interface 56327. One or more components of surgical element 56310, 56320, may be similar to and/or the same as features described with reference to 20282 of FIG. 5.

Surgical elements 56310, 56320, may be in communication with a patient 56302 (e.g., via sensor 56313, 56323, and/or output 56315, 56325, during a procedure). As an illustrative example, a ventilator (e.g., a surgical element 56310) and/or a pulse oximeter (e.g., surgical element 56320) may be attached to a patient during an open heart surgery. An HCP 56308 and/or a system 56330 may interact with one or more surgical elements 56310, 56320, to monitor the oxygen saturation of the patient 56302 (e.g., via the pulse oximeter), and/or determine a flow rate for a ventilator. In examples, the HCP 56308 may interact with the system 56330 and/or surgical elements 56310, 56320, via user interface 56317, 56327 (e.g., a graphical user interface (GUI), a knob, a button, a smart device a wearable electronic device, and/or the like).

Surgical element 56310, may be operated by an HCP 56308 and/or may operate autonomously (e.g., to perform a complementary task associated with a procedure). As an illustrative example, a surgical element 56310 may include a robotic surgical system, a navigation system, smart imaging systems, endoscopic and/or laparoscopic systems, harmonic scalpels, anesthesia machines, patient monitoring systems (pulse oximeters, blood pressure monitors, EKG monitors, EEG monitors, and/or the like), energy devices (e.g., electrosurgical units, laser surgery systems, and/or the like), infusion pumps, and/or the like.

In examples, surgical element 56310 may be wirelessly connected and/or physically connected (e.g., wired) to a network 56301, a system 56330, and/or another surgical element 56320. A surgical element 56310 may send/receive a dataflow from the system 56330 (e.g., to determine a boundary parameter). For example, a surgical element 56310 may send/receive a dataflow including a boundary parameter, an output parameter (e.g., for controlling output 56315, 56325), data generated by sensor 56313, 56323, data generated by the system 56330, training data, dataflow configuration information, data received by an HCP 56308, and/or the like.

As an illustrative example, a surgical element 56310 may be a heating blanket. The heating blanket may include a temperature measurement device (e.g., sensor 56313) and/or a heating element (e.g., output 56315). The heating blanket may be configured to energize the heating element, to maintain a patient's core body temperature during open heart surgery, based on a user input (e.g., from an HCP 56308) and/or based on a boundary parameter. The heating blanket may transmit a core body temperature measurement (e.g., as part of a dataflow) to the system 56330. A second surgical element 56320 may receive a boundary parameter (e.g., based on a determined relationship and/or based on an output parameter of surgical element 56310, 56320). The boundary parameter may include a setpoint and/or a limitation for a complementary task, such as an output power level for the heating blanket, a duration to maintain a power level, an indication that the heating blanket is to operate automatically, and/or the like. Advantageously, the heating blanket may receive an instruction to automatically control a patient's core body temperature based the determination of a task performed by a first surgical element 56310, such that the HCP 56308 may eliminate human error and/or optimize one or more tasks during a procedure.

Sensor 56313 may be any instrument, transducer, and/or the like, configured to measure one or more environmental conditions. In examples, sensor 56313 may measure physiological parameters of a patient 56302 such as oxygen saturation, blood pressure, respiratory rate, blood sugar, heart rate, a core body temperature and/or a hydration state and/or the like. Sensor 56313 may measure an environmental condition of an operating room (e.g., the operational environment 56300) such as a room temperature, the number of HCPs 56308 present during a procedure, the position of an HCP 56308 during a procedure, a humidity level, air quality, lighting levels, CO2 levels and/or the like. Sensor 56313 may measure one or more environmental conditions associated with a surgical element 56310 such as a current, voltage, a pressure, a force applied, a distance, a vibration, an orientation, a flow rate, a status, and/or the like. As an illustrative example, an HVAC system may include a temperature, humidity, and/or air quality sensor, a robotic surgical system may include a force and/or pressure sensor, a depth sensor, a temperature sensor, a blood flow and/or oxygen sensor, a pH sensor (e.g., to measure blood gas), an electrocardiogram (ECG) sensor, a position sensor (e.g., to measure the position of a robotic arm), and/or the like.

Sensor 56313 may send sensor data (e.g., information associated with a measured environmental condition) to a surgical element controller 56311. Sensor data may be sent based on dataflow configuration information. As described herein, dataflow configuration information may include an indication of a surgical element ID, an indication whether a dataflow is available, an update and/or acknowledgement of dataflow configuration information, a unit of measure, a communication protocol (RS-232, Ethernet, TCP/IP, Bluetooth, and/or the like), scheduling and/or frequency information (e.g., a time and/or frequency that sensor data is to be sent), a destination (e.g., port information associated with the surgical element controller 56311, 56321, and/or the system 56330), and/or security and/or access control credentials.

Output 56315 may be any number of devices and/or instruments as part of a surgical element 56310, that generate an output characteristic based on a received signal (e.g., based on an output parameter). In examples, output 56315 may include, among other components, motors and/or actuators (e.g., as part of a robotic device, a cutting tool, ventilators and/or the like), lighting output devices, GUls, cameras (e.g., as part of a borescope, IR camera, ultrasound camera, and/or the like), pumps (e.g., as part of smoke evacuation devices, suction devices, anesthesia delivery devices, IV infusion devices and/or the like), and/or lasers (e.g., as part of tissue ablation devices).

Output 56315 may receive data from a surgical element controller 56311. In examples, output 56315 may adjust, based on data received from a surgical element controller 56311, an output characteristic such as the speed of a cutting tool, the power intensity of an electrosurgical tool, a flow rate for an infusion pump, a position of a robotic arm, and/or the like. Although one output 56315 is included in surgical element 56310, it is to be understood that there may be multiple outputs, each receiving data and generating an output characteristic. As an illustrative example, a robotic system may include multiple outputs 56315 generating any number of outputs including positioning a robot, performing a measurement, capturing an image, creating an incision, and/or the like.

A surgical element 56310 may include a user interface 56317. User interface service 56213 may include buttons, switches, levers, sliders, touchscreens, a GUI, a user device, and/or the like, to enable the surgical element 56310 to interact with the user (e.g., an HCP 56308). User interface 56317 may display information received from surgical element controller 56311 and/or from the system 56330. User interface 56317 may generate a GUI to display data from one or more components of operating environment 56300. In examples, user interface 56317 may display data associated with a dataflow (e.g., a physiological parameter of a patient), an indication of a relationship, a boundary parameter, an output parameter, a task, a complementary task, and/or the like. A user interface 56317 may request a user input from an HCP 56308. In examples, a user interface 56317 may display a request for information associated with a surgical element 56310 (e.g., an identification, a function, capability, an output, an input, and/or the like), and/or a request associated with a task (e.g., to approve a determined boundary parameter, to amend a boundary parameter, to select a task and/or a complementary task, to confirm a relationship, and/or the like).

Surgical element 56310 may include a surgical element controller 56311. Surgical element controller 56311 may receive sensor data from sensor 56313. Surgical element controller 56311 may transmit data to the system 56330, output 56315, and/or user interface 56317 (including and/or indicating an output parameter). A surgical element controller 56311 may communicate a dataflow (e.g., sensor data, a boundary parameter, an output parameter, dataflow configuration information, and/or the like) to/from a system 56330. In examples, a surgical element controller 56311 may create and/or send a first and/or second dataflow to/from a system 56330. In examples, surgical element controller 56311 may receive, as part of a dataflow, a boundary parameter as described herein. surgical element controller 56311 may perform a task (e.g., a complementary task) based on a received boundary parameter.

It is to be understood that surgical element 56320 and/or one or more associated components (e.g., sensor 56323, output 56325, user interface 56327, and/or surgical element controller 56321) may include the same and/or similar interactions, features, functionalities, and/or the like, as described herein with reference to surgical element 56310 and/or one or more components of surgical element 56310 (e.g., sensor 56313, output 56315, user interface 56317, and/or surgical element controller 56311). Surgical element 56320 may be a second surgical element (e.g., separate from surgical element 56310). In examples, communication between one or more components as described with reference to surgical element 56310 may be the same and/or similar to one or more components of surgical element 56320. As an illustrative example, surgical element 56320 may send and/or receive a dataflow to/from a system 56330 and/or a surgical element 56310, including sensor data, dataflow configuration information, an output parameter, and/or the like.

An operational environment 56300 may include a network 56301. The network 56301 may include one or more communications networks, such as the Internet. The network 56301 may be any combination of local area network ("LAN") and/or a wireless area network ("WAN") or the like. Accordingly, various components of the operational environment 56300, including the system 56330, surgical element 56310, 56320, and/or HCP 56308 (e.g., via a user device) may communicate with one another directly or indirectly via any appropriate communications links and/or networks, such as network 56301 (e.g., one or more communications links, one or more computer networks, one or more wired or wireless connections, the Internet, and/or the like).

### Example Routine for Determining an Optimized Boundary Parameter

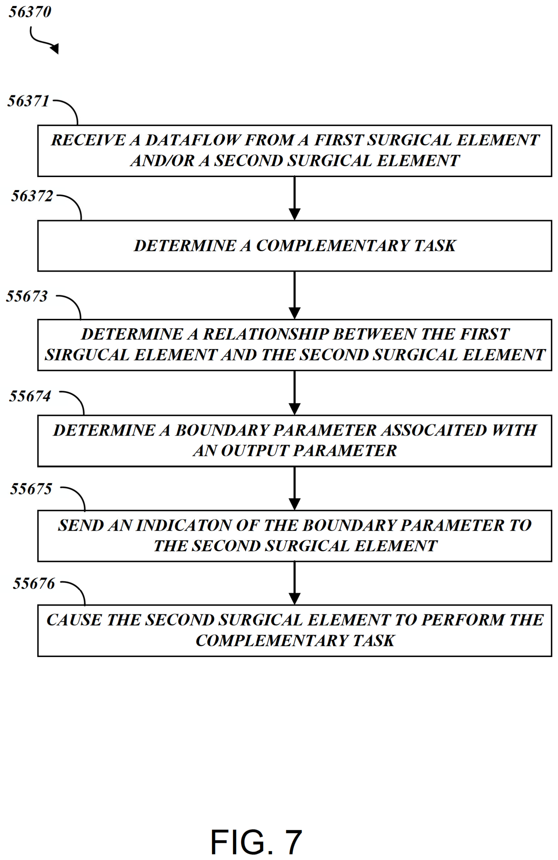

FIG. 7 illustrates an example routine 56370 for determining an optimized boundary parameter. Example routine 56370 may be executed by, for example, a system 56330 (e.g., dataflow analyzer 56332) of operational environment 56300. The example routine 56370 begins at block 56371.

At block 56371, a dataflow analyzer 56332 may receive a dataflow from a first surgical element and/or a second surgical element 56310, 56320. As described herein the dataflow analyzer 56332 may receive a dataflow including data generated by and/or associated with the system 56330, a surgical element 56310, 56320, a patient 56302, and/or an HCP 56308. For example, a dataflow may include a boundary parameter, an output parameter (e.g., for controlling output 56315, 56325), data generated by sensor 56313, 56323, data generated by the system 56330, training data, dataflow configuration information, data received by an HCP 56308, and/or historical data. In examples, a boundary parameter may indicate an adjustment and/or a constraint associated with the second smart device during the complementary task. An output parameter may indicate a control variable setpoint, a current and/or voltage, an instruction, and/or a power level. Data generated by sensor 56313, 56323 may include a signal indicating one or more physiological parameters of a patient, an environmental characteristic of the operating room, and/or one or more characteristics of surgical element 56310, 56320.

In examples, data generated by the system 56330 may include a procedure plan, morbidity data associated with one or more patients, an HCP 56308 skill level, preferred tools used by an HCP 56308, a task, supplies, whether a surgical element 56310, 56320 is available for a procedure, staff workflow, and/or the like. Data generated by the system 56330 may include an indication of a relationship between a first surgical element 56310 and a second surgical element 56320, an indication of a task and/or complementary task that may be automated, and/or or a threshold associated with a physiological parameter of a patient, a threshold associated with a boundary parameter, and/or the like.

In examples, training data may include an indication of a boundary parameter, an output parameter, data generated by sensor 56313, 56323, data generated by the system 56330, data received by an HCP 56308, historical data, and/or dataflow configuration information used to train an ML model. In examples, dataflow configuration information may include an indication of a surgical element ID, an indication whether a dataflow is available, a unit of measure, a communication protocol as described herein, scheduling and/or frequency information, a destination, and/or security and/or access control credentials. In examples, historical data may include past information associated with a procedure. For example, historical data may include a dataflow. Data analyzer 65332 may use historical data to, among other things, determine a relationship between surgical elements 56310, 56320 as described herein.

In examples, data received from an HCP 56308 may include an indication of a procedure, a task, a complementary task, a relationship, a threshold, an instruction to automate a task, and/or the like. Dataflow analyzer 56332 may receive a user input from and/or generate an output to an HCP 56308 (e.g., via a dataflow sent to user interface 56317, 56327). In examples, dataflow analyzer 56332 may receive sensor data from a first and/or second surgical element 56310, 56320, and send an indication of an alert to the HCP 56308 (e.g., via the dataflow).

At block 56372, a dataflow analyzer 56332 may determine a complementary task. As described herein, a complementary task may be associated with a first task during a procedure. In examples, a complementary task may be a sub-task, a supportive task, a contributing task, a cooperative task, an ancillary task, a supplementary task, and/or a task related to another task. A complementary task may be performed along with a first task to achieve a target outcome. As an illustrative example, a first task may include applying energy to ablate tissue (e.g., via a first surgical element 56310). The dataflow analyzer 56332 may determine that a complementary task includes applying tension to the tissue (e.g., via a second surgical element 56320 operating autonomously) to achieve a target energy density while the first surgical element 56310 applies energy to the tissue.

At block 56373, a dataflow analyzer 56332 may determine a relationship between the first surgical element 56310 and the second surgical element 56320. As described herein, dataflow analyzer 56332 may receive a user input form an HCP 56308, including a procedure, a task, a complementary task, and/or the like (e.g., to be performed autonomously by a surgical element 56310, 56320). A relationship may be determined and/or inferred based on, for example, compatible sensors (e.g., sensor 56313, 56323) between surgical elements 56310, 56320, a determined dataflow that may be needed to resolve an issue during a procedure, and/or based on a user input (e.g., by an HCP 56308 via a user interface 56317, 56327). As an illustrative example, a dataflow analyzer 56332 may receive a first dataflow from a laparoscopic tool (e.g., a dataflow indicating a measured temperature of an insufflated cavity via sensor 56313) and a second dataflow from a heating pad (e.g., a dataflow indicating a measured temperature near an insufflated cavity via sensor 56323). The dataflow analyzer 56332 may determine, based on configuration information, sensor data, and/or an output parameter associated with each dataflow, that the two dataflows are related (e.g., a change detected by a first temperature sensor of a second surgical element 56320 may be related to a response and/or a change in an output parameter for a first surgical element 56310). In examples, sensor data may be related if, for example, the sensor data from a first and second surgical element 56310, 56320, share a similar unit of measure, measure a similar physiological parameter of a patient's body, are proximate to one another during a procedure, and/or the like.

After the dataflow analyzer 56332 determines that one or more aspects of surgical elements 56310, 56320 are related, the dataflow analyzer 56332 may store an indication of the relationship in, for example, historical datastore 56334 (e.g., in a LUT defining the one or more relationships). Additionally and/or alternatively, dataflow analyzer 56332 provide training data (e.g., including historical data) to ML model trainer 56336, to train an ML model based on one or more relationships.

At block 56374, a dataflow analyzer 56332 may determine a boundary parameter associated with an output parameter. As described herein, a boundary parameter may indicate a spatial boundary, a force, a speed and/or acceleration, a proximity, a range of motion, a tissue type, an image, a user controlled limit, and/or the like. As an illustrative example, a spatial boundary parameter may enable a second surgical element 56320 to maintain a safe distance to avoid interference or a collision with a first surgical element 56310. A boundary parameter (e.g., a force boundary parameter) may limit excessive pressure and/or strain on tissue to prevent tissue damage or maintain a target energy density (e.g., during a moving ablation procedure). A speed and/or acceleration boundary may limit how quickly a surgical device may move (e.g., limit an output parameter such as the speed of a motor while moving through high-risk areas). A proximity boundary may limit motion to prevent unintentional contact with the patient 56302, surgical tools, an HCP 56308, and/or the like. A tissue-type boundary parameter may limit an action based on a tissue characteristic (e.g., based on tissue density, elasticity, high-vascularity, and/or how delicate the tissue is).

A dataflow analyzer 56332 may determine a second boundary parameter based on a first boundary parameter. As an illustrative example, a dataflow analyzer 56332 may determine a tissue-type boundary parameter (e.g., the dataflow analyzer 56332 may limit an action associated with a complementary task based on a determined tissue density, elasticity, high-vascularity, or how delicate the tissue is). The tissue-type boundary parameter may be used to determine a spatial boundary parameter and/or a velocity boundary parameter (e.g., to prevent unintentional contact or reduce the likelihood of tissue damage). A dataflow analyzer 56332 may use an image boundary parameter to adjust a spatial boundary parameter and/or adjust a range of motion (e.g., to limit the movement of a catheter and/or the like).

In examples, a boundary parameter may be associated with an operating envelope for a second surgical element 56320. The boundary parameter may indicate an adjustment to an operating envelope of a second surgical element 56320 based on an action associated with a first surgical element 56310.

In examples, a boundary parameter may include a safety envelope. A dataflow analyzer 56332 may determine a safety envelope for a patient 56302 based on a dataflow, historical data, and/or a user input from an HCP 56308. For example, dataflow analyzer 56332 may determine that a task and/or a complementary task may be performed during a first time based on a determined boundary parameter (e.g., the task and/or complementary task may be performed within a time period associated with a determined safety envelope). As an illustrative example, a dataflow analyzer 56332 may determine a blood pressure range. A task and/or a complementary task may be performed while the patient's blood pressure is within the blood pressure range (e.g., within the safety envelope). In examples, for patient's susceptible to hypothermia, a dataflow analyzer 56332 may determine a safety envelope that may maintain a body temperature within a range during a task and/or complementary task. In examples, if the safety envelope threshold is satisfied (e.g., a parameter falls outside a range and/or the like), the dataflow analyzer 56332 may determine a boundary parameter that reduces an action associated with a complementary task, increases the level of automation to fully automate a task, and/or stops a complementary task, and/or alerts the HCP 56308.

A dataflow analyzer 56332 may determine a boundary parameter based on a complementary task. A complementary task may be associated with a first task during a procedure. In examples, a complementary task may be a sub-task, a supportive task, a contributing task, a cooperative task, an ancillary task, a supplementary task, and/or a task related to another task. A complementary task may be performed along with a first task to achieve a target outcome. As an illustrative example, a first task may include applying energy to ablate tissue (e.g., via a first surgical element 56310), while a complementary task may include applying tension to the tissue (e.g., via a second surgical element 56320 operating autonomously) to achieve a target energy density during the application of energy. In examples, the boundary parameter may include an instruction to perform a complementary task in a synchronized motion based on a movement of a first surgical element 56310 (e.g., while the first surgical element 56310 performs a first task). For example, the boundary parameter may instruct the second surgical element 56320 to vary an amount of force applied to tissue relative to the amount of force applied by the HCP 56308 (e.g., via the first surgical element 56310) and/or relative to the energy output determined by the HCP 56308, to achieve a target energy density.

Dataflow analyzer 56332 may determine a boundary parameter based on a physiological parameter of a patient (e.g., received from surgical element 56310, 56320). For example, the dataflow analyzer 56332 may determine a boundary parameter based on a perfusion level, an electrophysiological signal, a blood pressure, a heart rate, a respiratory rate, the temperature of tissue, SpO2, CO2 levels, electroencephalogram (EEG) readings, and/or the like. As an illustrative example, dataflow analyzer 56332 may receive a dataflow including an indication of a tissue temperature, and determine a boundary parameter that limits the movement of a second surgical element 56320 during ablation (e.g., if a tissue's temperature exceeds a threshold, the boundary parameter may limit a robot's movement proximate to the tissue to reduce the temperature of the tissue). As a second illustrative example, if a patient's SpO2 level satisfies a threshold, dataflow analyzer 56332 may determine a boundary parameter that limits a robot's movement in proximity to the patient's lungs and/or airways.

At block 56375, a dataflow analyzer 56332 may send an indication of the boundary parameter to the second surgical element 56320. The indication may of the boundary parameter may be sent as part of, for example, a dataflow between the second surgical element 56320 and the dataflow analyzer 56332.

At block 56376, a dataflow analyzer 56332 may cause the second surgical element 56320 to perform the complementary task. The complementary task may be performed based on the boundary parameter. In examples, the boundary parameter may include an instruction to perform a complementary task in a synchronized motion based on a movement of a first surgical element 56310 (e.g., while the first surgical element 56310 performs a first task based on a user input). For example, the boundary parameter may instruct the second surgical element 56320 to vary an amount of force applied to tissue relative to the amount of force applied by the HCP 56308 (e.g., via the first surgical element 56310) and based on the energy output determined by the HCP 56308, to achieve a target energy density. After the dataflow analyzer 56332 causes the second surgical element 56320 to perform the complementary task, the routine 56370 may end.

An illustrative example described herein may provide an (e.g., bounded fully) autonomous operation of a first system (e.g., a first surgical element) in cooperation with an operation of another system controlled directly by the surgeon (e.g., a second surgical element).

A procedure may include an ablative remodeling of the heart to treat AFIB. An automatic RF catheter motion control system (e.g., a second surgical element controlled by the surgeon) may ablate a predefined area of tissue, with predefined depth, pressure or power intensity levels. The surgeon may provide the area of intended treatment after mapping of the heart and/or provide the depth of the ablation desired.

The system (e.g., system 56330) may display a mapped path of the catheter motion and may receive confirmation that a robot (e.g., a first surgical element) sweeps the catheter through the path maintaining the pressure and/or energy balance while compensating for the movement of the heart in the process. In examples, bounding of the automation (e.g., bounding motions of the surgeon) enables the smart system (e.g., 56330) to actively interact with and support surgeon controlled motions of instruments while including some limits to the motion to prevent the cooperative or antagonistic actions or forces from causing collateral damage, or inadvertently colliding with nearby structures (e.g., the system 56330 may determine an automation assistance parameter to bound movement of the robot that are requested by the surgeon).

To create the sufficient contact and/or pressure for a moving ablation system to cause the cautery and/or ablation (e.g., which is dependent on pressure, power and mode of energy) the user (e.g., surgeon) may control an ablative electrode. Using the ablative electrode, the user may define the electrode's path and/or pushing force capability (e.g., to create the needed energy density to produce the sufficient tissue welding or tissue death that the assisting system (e.g., the robot) would have to provide) in order to maintain the needed energy density. In examples, the heart may be a sensitive and interconnected structure that could easily be injured inadvertently if too much force or too much differential motion is applied. The robot may monitor the surgical image of the system 56330 for adjacent structures and/or may monitor its own applied forces relative to the force applied from the user. The sum of the forces relative to the fixation of the heart to the surrounding anatomy may be limited to the applicable force and motions (e.g., the robot may, based on the surgeons movement of an electrode, apply more and/or less force to maintain contact during tissue modification, resulting in an operational environment 56300 having less and/or more automation, depending on the actions of the surgeon).

### Example Aspects Related to Determining a Boundary Parameter for a Procedure

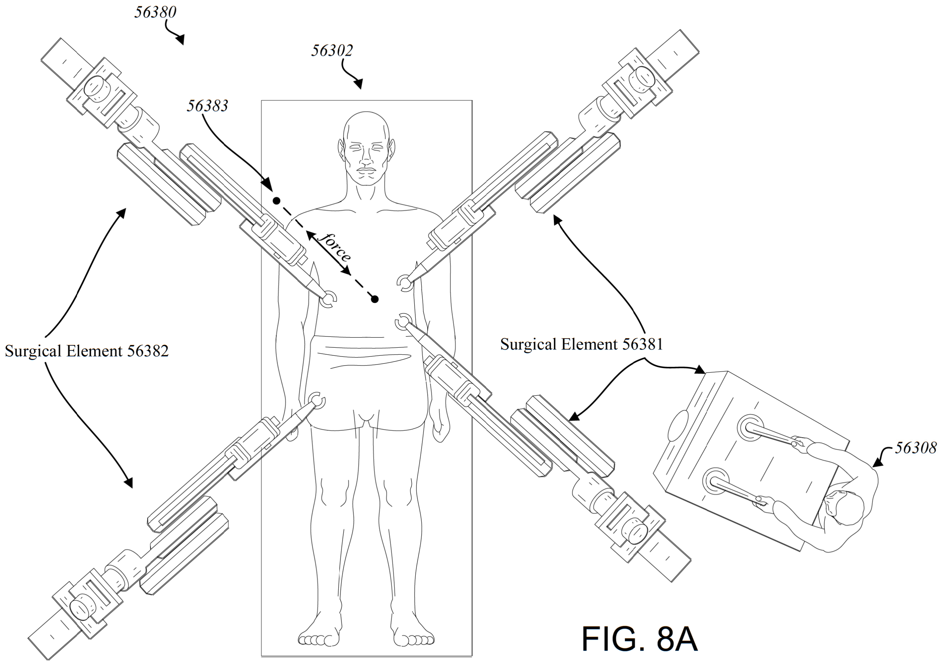

FIG. 8A is an example operational environment 56380 where a surgical element 56381 may be operated by an HCP 56308 and/or surgical element 56382 may operate autonomously as a moving ablation system.

Surgical element 56381 may include features similar to and/or the same as surgical element 56310 of operational environment 56300. For example, the console may be similar to a user interface 56317 of operational environment 56300. Surgical element 56381 and/or 56382 may include one or more components and/or features associated with a surgical computing system (e.g., similar to system 56330 of operational environment 56300). The operational environment 56380 may include a boundary parameter 56383 determined by surgical element 56381 and/or surgical element 56382.

As illustrated, surgical element 56381 includes a robot system operated manually by an HCP 56308. Surgical element 56381 includes an electrode defining a path to ablate tissue, and/or a means to generate a pushing force. The electrode and/or pushing force may be used to create a target energy density, to produce sufficient tissue welding or tissue death.

Surgical element 56382 may operate autonomously (e.g., as an assisting system to surgical element 56381). Surgical element 56382 may execute a complementary task in response to one or more inputs from the HCP 56308 via surgical element 56381. As illustrated, surgical element 56382 may provide reactionary and/or additive complementary forces or displacement (e.g., via boundary parameter 56383) to maintain a target energy density (e.g., based on determined boundary parameter). The total force that may be applied by the second surgical element 56382 may be limited based on the boundary parameter 56383 (e.g., the limitation may be indicated with a solid line and arrows). Whereas the total amount of force that may be applied by the surgical element 56382 may be greater (e.g., as illustrated with a dotted line terminating in two endpoints).

The determined boundary parameter may include an instruction to apply a force and/or tension (e.g., via surgical element 56382) to tissue relative to the force applied by the HCP 56308 via surgical element 56381 (e.g., an autonomous robotic system may perform a complementary task in a synchronized motion, based on the HCP-controlled robotic system). Since the heart is a sensitive and interconnected structure that could easily be injured inadvertently if too much force or too much differential motion is applied, the surgical element 56382 may, for example, monitor a surgical image for adjacent structures and/or the force applied by the surgical element 56382 relative to the force applied form the HCP 56308 (e.g., via surgical element 56381) and/or the sum of forces (e.g., of surgical element 56381, 56382) relative to the fixation of the heart to the surrounding anatomy, to limit the total force applied and/or to limit a total motion (e.g., to achieve a target energy density during the moving ablation).

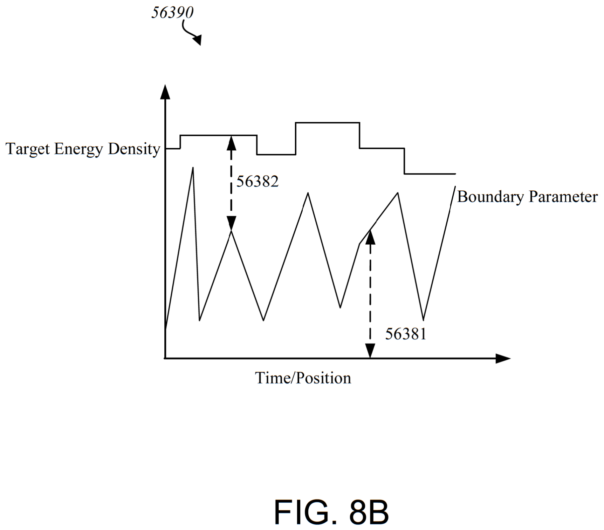

FIG. 8B is an graph 56390 illustrating an example boundary parameter that may be determined by a surgical element 56381 and/or 56382, to perform a complementary task of FIG. 8A. As illustrated, a system (e.g., as part of surgical element 56381 and/or 56382) determines a target energy density as a function of time, position, and/or the like during a moving ablation procedure. The target energy density may change based on one or more factors such as the type of tissue ablated (e.g., tissue characteristics), the location of tissue ablated, the speed of the energy device (e.g., an output parameter), a change in contact angle of the energy device, energy output, and/or the like. The boundary parameter may be determined based on characteristics of the first surgical element 56381 and/or the second surgical element 56382. For example, the boundary parameter may indicate and/or limit a force, tension, pressure, energy output, energy device angle, speed, and/or the like.

As an example, the target energy density may be achieved based on a determined sum of forces. The boundary parameter may indicate a limit (e.g., or a setpoint) of force that may be applied by the second surgical element 56382 (e.g., operating autonomously) based on a force applied by the first surgical element 56381 (e.g., operating by an input from an HCP 56308). Although graph 56390 is described with an example boundary parameter related to a force, this is not meant to be limiting, as multiple inputs (e.g., a force, tension, pressure, energy output, energy device angle, speed, and/or the like) may be considered to determine a boundary parameter to achieve the target energy density (e.g., to achieve synchronized motion of the second surgical element 56382 based on an action of the first surgical element 56381).

The following list of numbered Examples forms part of the present disclosure:
1. A system for rendering assistance to a user based on an adaptive recognition of abilities during a procedure, the system comprising:
   a processor configured to:
   receive a first dataflow from a first surgical element, wherein the first dataflow indicates a first output parameter associated with a first task;
   receive a second dataflow from a second surgical element, wherein the second dataflow indicates a second output parameter associated with a complementary task, and wherein the second surgical element is configured to perform the complementary task based on the first task;
   determine a relationship between the first surgical element and the second surgical element based on the first dataflow and the second dataflow, wherein the relationship indicates that the complementary task is associated with the first task;
   determine a boundary parameter associated with the second output parameter based on the first output parameter and the relationship, wherein the boundary parameter indicates an adjustment to the second surgical element during the complementary task;
   send an indication of the boundary parameter to the second surgical element; and
   cause the second surgical element to perform the complementary task based on the boundary parameter.
2. The system of Example 1,
   wherein the first surgical element is a first surgical robot configured to be controlled by a user;
   wherein the first task is associated with applying radio frequency (RF) energy to tissue of a patient during tissue ablation;
   wherein the second surgical element is a second surgical robot configured to be controlled autonomously;
   wherein the complementary task is associated with maintaining a tension applied to tissue to secure the tissue during the first task;
   wherein the boundary parameter is an energy density applied to the tissue during the tissue ablation; and
   wherein the processor is further configured to:
      determine the tension to be applied by the second surgical robot during an application RF energy to the tissue by the first surgical robot, wherein the tension to be applied maintains the energy density applied to the tissue.
3. The system of Example 1, wherein the processor is further configured to:
   receive a physiological parameter of a patient from the first dataflow;
   determine a second boundary parameter associated with the second output parameter;
   determine that the physiological parameter of the patient satisfies a threshold;
   select the second boundary parameter based on a determination that the physiological parameter satisfied the threshold;
   send an indication of the second boundary parameter to the second surgical element; and
   cause the second surgical element to perform the complementary task based on the boundary parameter and the second boundary parameter.
4. The system of Example 1, wherein the first surgical element is configured to execute the first task based on an input from a user, and wherein the second surgical element is configured to execute the complementary task autonomously.
5. The system of Example 1, wherein the processor is further configured to:
   determine that the second output parameter satisfies a threshold, wherein the threshold is associated with the boundary parameter; and
   send an alert message to the first surgical element, wherein the alert message indicates that the output parameter exceeds the boundary parameter.
6. The system of Example 1, wherein the second surgical element is configured to perform the complementary task in a synchronized motion based on a movement of the first surgical element while performing the first task.
7. The system of Example 1, wherein the relationship between the first surgical element and the second surgical element is determined based on a lookup table.
8. The system of Example 1, wherein the processor is further configured to:
   send the first dataflow, the second dataflow, an indication of the first task, and an indication of the complementary task as an input to a machine learning (ML) model; and
   receive, as a response form the ML model, the boundary parameter associated with the second output parameter.
9. A method for rendering assistance to a user based on an adaptive recognition of abilities during a procedure, the method comprising:
   receiving a first dataflow from a first surgical element, wherein the first dataflow indicates a first output parameter associated with a first task;
   receiving a second dataflow from a second surgical element, wherein the second dataflow indicates a second output parameter associated with a complementary task, and wherein the second surgical element is configured to perform the complementary task based on the first task;
   determining a relationship between the first surgical element and a second surgical element based on the first dataflow and the second dataflow, wherein the relationship indicates that the complementary task is associated with the first task;
   determining a boundary parameter associated with the second output parameter based on the first output parameter and the relationship, wherein the boundary parameter indicates an adjustment to the second surgical element during the complementary task;
   sending an indication of the boundary parameter to the second surgical element; and
   causing the second surgical element to perform the complementary task based on the boundary parameter.
10. The method of Example 9,
   wherein the first surgical element is a first surgical robot configured to be controlled by a user;
   wherein the first task is associated with applying radio frequency (RF) energy to tissue of a patient during tissue ablation;
   wherein the second surgical element is a second surgical robot configured to be controlled autonomously;
   wherein the complementary task is associated with maintaining a tension applied to tissue to secure the tissue during the first task;
   wherein the boundary parameter is an energy density applied to the tissue during the tissue ablation; and
   wherein the method further comprises:
      determining the tension to be applied by the second surgical robot during an application RF energy to the tissue by the first surgical robot, wherein the tension to be applied maintains the energy density applied to the tissue.
11. The method of Example 9, further comprising:
   receiving a physiological parameter of a patient from the first dataflow;
   determining a second boundary parameter associated with the second output parameter;
   determining that the physiological parameter of the patient satisfies a threshold;
   selecting the second boundary parameter based on a determination that the physiological parameter satisfied the threshold;
   sending an indication of the second boundary parameter to the second surgical element; and
   causing the second surgical element to perform the complementary task based on the boundary parameter and the second boundary parameter.
12. The method of Example 9, wherein the first surgical element is configured to execute the first task based on an input from a user, and wherein the second surgical element is configured to execute the complementary task autonomously.
13. The method of Example 9, wherein the method further comprises:
   determining that the second output parameter satisfies a threshold, wherein the threshold is associated with the boundary parameter; and
   sending an alert message to the first surgical element, wherein the alert message indicates that the output parameter exceeds the boundary parameter.
14. The method of Example 9, wherein the second surgical element is configured to perform the complementary task in a synchronized motion based on a movement of the first surgical element while performing the first task.
15. The method of Example 9, wherein the method further comprises:
   sending the first dataflow, the second dataflow, an indication of the first task, and an indication of the complementary task as an input to a machine learning (ML) model; and
   receiving, as a response form the ML model, the boundary parameter associated with the second output parameter.
16. A system for rendering assistance to a user based on an adaptive recognition of abilities during a procedure, the system comprising:
   a first surgical element configured to send a first dataflow;
   a second surgical element configured to receive a second dataflow; and
   a processor configured to:
      receive the first dataflow, wherein the first dataflow indicates an output parameter associated with a first task and a complementary task;
      determine a relationship between the first surgical element and the second surgical element, wherein the relationship indicates that the complementary task is associated with the first task;
      determine a boundary parameter associated with the second surgical element based on the output parameter and the relationship;
      send an indication of the boundary parameter to the second surgical element; and
      cause the second surgical element to perform the complementary task based on the boundary parameter.
17. The system of Example 16, wherein the processor is further configured to:
   receive a physiological parameter of a patient from the first dataflow;
   determine a second boundary parameter associated with the second surgical element;
   determine that the physiological parameter of the patient satisfies a threshold;
   select the second boundary parameter based on a determination that the physiological parameter satisfied the threshold;
   send an indication of the second boundary parameter to the second surgical element; and
   cause the second surgical element to perform the complementary task based on the boundary parameter and the second boundary parameter.
18. The system of Example 16, wherein the first surgical element is configured to execute the first task based on an input from a user, and wherein the second surgical element is configured to execute the complementary task autonomously.
19. The system of Example 16, wherein the processor is further configured to send an alert message to the first surgical element, wherein the alert message indicates that the output parameter exceeds the boundary parameter.
20. The system of Example 16, wherein the processor is further configured to:
   send the first dataflow and an indication of the first task as an input to a machine learning (ML) model; and
   receive, as a response form the ML model, the boundary parameter.

## Claims

1. A system for rendering assistance to a user based on an adaptive recognition of abilities during a procedure, the system comprising:
a processor configured to:
receive a first dataflow from a first surgical element, wherein the first dataflow indicates a first output parameter associated with a first task;
receive a second dataflow from a second surgical element, wherein the second dataflow indicates a second output parameter associated with a complementary task, and wherein the second surgical element is configured to perform the complementary task based on the first task;
determine a relationship between the first surgical element and the second surgical element based on the first dataflow and the second dataflow, wherein the relationship indicates that the complementary task is associated with the first task;
determine a boundary parameter associated with the second output parameter based on the first output parameter and the relationship, wherein the boundary parameter indicates an adjustment to the second surgical element during the complementary task;
send an indication of the boundary parameter to the second surgical element; and
cause the second surgical element to perform the complementary task based on the boundary parameter.

2. A computer-readable medium comprising instructions which, when executed by a computer, cause the computer to:
receive a first dataflow from a first surgical element, wherein the first dataflow indicates a first output parameter associated with a first task;
receive a second dataflow from a second surgical element, wherein the second dataflow indicates a second output parameter associated with a complementary task, and wherein the second surgical element is configured to perform the complementary task based on the first task;
determine a relationship between the first surgical element and the second surgical element based on the first dataflow and the second dataflow, wherein the relationship indicates that the complementary task is associated with the first task;
determine a boundary parameter associated with the second output parameter based on the first output parameter and the relationship, wherein the boundary parameter indicates an adjustment to the second surgical element during the complementary task;
send an indication of the boundary parameter to the second surgical element; and
cause the second surgical element to perform the complementary task based on the boundary parameter.

3. The system of claim 1 or computer-readable medium of claim 2,
wherein the first surgical element is a first surgical robot configured to be controlled by a user;
wherein the first task is associated with applying radio frequency (RF) energy to tissue of a patient during tissue ablation;
wherein the second surgical element is a second surgical robot configured to be controlled autonomously;
wherein the complementary task is associated with maintaining a tension applied to tissue to secure the tissue during the first task;
wherein the boundary parameter is an energy density applied to the tissue during the tissue ablation; and
wherein the processor is further configured to, or the instructions further cause the computer to:
determine the tension to be applied by the second surgical robot during an application RF energy to the tissue by the first surgical robot, wherein the tension to be applied maintains the energy density applied to the tissue.

4. The system or computer-readable medium of any preceding claim, wherein the processor is further configured to, or the instructions further cause the computer to:
receive a physiological parameter of a patient from the first dataflow;
determine a second boundary parameter associated with the second output parameter;
determine that the physiological parameter of the patient satisfies a threshold;
select the second boundary parameter based on a determination that the physiological parameter satisfied the threshold;
send an indication of the second boundary parameter to the second surgical element; and
cause the second surgical element to perform the complementary task based on the boundary parameter and the second boundary parameter.

5. The system or computer-readable medium of any preceding claim, wherein the first surgical element is configured to execute the first task based on an input from a user, and wherein the second surgical element is configured to execute the complementary task autonomously.

6. The system or computer-readable medium of any preceding claim, wherein the processor is further configured to, or the instructions further cause the computer to:
determine that the second output parameter satisfies a threshold, wherein the threshold is associated with the boundary parameter; and
send an alert message to the first surgical element, wherein the alert message indicates that the output parameter exceeds the boundary parameter.

7. The system or computer-readable medium of any preceding claim, wherein the second surgical element is configured to perform the complementary task in a synchronized motion based on a movement of the first surgical element while performing the first task.

8. The system or computer-readable medium of any preceding claim, wherein the relationship between the first surgical element and the second surgical element is determined based on a lookup table.

9. The system or computer-readable medium of any preceding claim, wherein the processor is further configured to, or the instructions further cause the computer to:
send the first dataflow, the second dataflow, an indication of the first task, and an indication of the complementary task as an input to a machine learning (ML) model; and
receive, as a response from the ML model, the boundary parameter associated with the second output parameter.

10. A system for rendering assistance to a user based on an adaptive recognition of abilities during a procedure, the system comprising:
a first surgical element configured to send a first dataflow;
a second surgical element configured to receive a second dataflow; and
a processor configured to:
receive the first dataflow, wherein the first dataflow indicates an output parameter associated with a first task and a complementary task;
determine a relationship between the first surgical element and the second surgical element, wherein the relationship indicates that the complementary task is associated with the first task;
determine a boundary parameter associated with the second surgical element based on the output parameter and the relationship;
send an indication of the boundary parameter to the second surgical element; and
cause the second surgical element to perform the complementary task based on the boundary parameter.

11. A computer-readable medium comprising instructions which, when executed by a computer, cause the computer to:
receive a first dataflow from a first surgical element, wherein the first dataflow indicates an output parameter associated with a first task and a complementary task;
determine a relationship between the first surgical element and a second surgical element, wherein the relationship indicates that the complementary task is associated with the first task;
determine a boundary parameter associated with the second surgical element based on the output parameter and the relationship;
send an indication of the boundary parameter to the second surgical element; and
cause the second surgical element to perform the complementary task based on the boundary parameter.

12. The system of claim 10 or computer-readable medium of claim 11, wherein the processor is further configured to, or the instructions further cause the computer to:
receive a physiological parameter of a patient from the first dataflow;
determine a second boundary parameter associated with the second surgical element;
determine that the physiological parameter of the patient satisfies a threshold;
select the second boundary parameter based on a determination that the physiological parameter satisfied the threshold;
send an indication of the second boundary parameter to the second surgical element; and
cause the second surgical element to perform the complementary task based on the boundary parameter and the second boundary parameter.

13. The system or computer-readable medium of any of claims 10 to 12, wherein the first surgical element is configured to execute the first task based on an input from a user, and wherein the second surgical element is configured to execute the complementary task autonomously.

14. The system or computer-readable medium of any of claims 10 to 13, wherein the processor is further configured to, or the instructions further cause the computer to:
send an alert message to the first surgical element, wherein the alert message indicates that the output parameter exceeds the boundary parameter.

15. The system or computer-readable medium of any of claims 10 to 14, wherein the processor is further configured to, or the instructions further cause the computer to:
send the first dataflow and an indication of the first task as an input to a machine learning (ML) model; and
receive, as a response from the ML model, the boundary parameter.
